# EUROPÄISCHE PATENTSCHRIFT

(11) **EP 3 438 112 B1**
(45) Veröffentlichungstag und Bekanntmachung des Hinweises auf die Patenterteilung: **04.12.2019**
(21) Anmeldenummer: 17183989.7
(22) Anmeldetag: 31.07.2017
(51) Int. Cl.: C07F 9/50

(54) **LIGANDEN MIT EINER O-P-O-BRÜCKE ZWISCHEN ZWEI ANTHRACENTRIOLBAUSTEINEN**
LIGANDS HAVING AN O-P-O BRIDGE BETWEEN TWO ANTHRACENTRIOL BUILDING BLOCKS
LIGANDS AVEC UN PONT O-P-O ENTRE DEUX ÉLÉMENTS D'ANTHRACÈNE

(43) Veröffentlichungstag der Anmeldung: 06.02.2019
(73) Patentinhaber: Evonik Degussa GmbH, 45128 Essen (DE)
(72) Erfinder: BÖRNER, Armin, 18059 Rostock (DE); FRANKE, Robert, 45772 Marl (DE); SELENT, Detlef, 18059 Rostock (DE)
(74) Vertreter: Evonik Patent Association

(56) Entgegenhaltungen:
- WO-A1-2008/088495
- WO-A2-02/22261
- ROBERT FRANKE ET AL: "Applied Hydroformylation", CHEMICAL REVIEWS, Bd. 112, Nr. 11, 14. November 2012 (2012-11-14), Seiten 5675-5732, XP55091930, ISSN: 0009-2665, DOI: 10.1021/cr3001803

## Beschreibung

Die Erfindung betrifft Liganden mit einer O-P-O-Brücke zwischen zwei Anthracentriolbausteinen.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

Der Erfindung lag die Aufgabe zugrunde Liganden bereitzustellen, welche zwei Anthracentriolbausteinen aufweisen, und welche als Liganden in der Hydroformylierung eingesetzt werden können, und dort eine sehr gute Gesamtausbeute an Aldehyd liefern.

Gelöst wird die Aufgabe durch eine Verbindung gemäß Anspruch 1.

Verbindung der Formel (**I**): 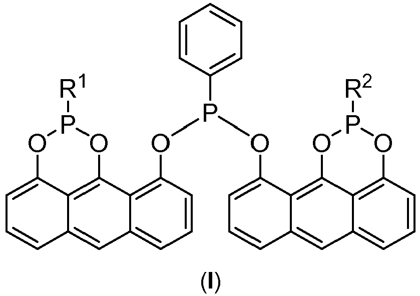 wobei
R¹, R² ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl;
wobei die genannten Alkylgruppen und Arylgruppen wie folgt substituiert sein können: substituierte -(C₁-C₁₂)-Alkylgruppen, substituierte -O-(C₁-C₁₂)-Alkyl, substituierte -(C₆-C₂₀)-Aryl, substituierte -O-(C₆-C₂₀)-Aryl können einen oder mehrere Substituenten aufweisen; die Substituenten sind unabhängig voneinander ausgewählt aus: -(C₁-C₁₂)-Alkyl, -O-(C₁-C₁₂)-Alkyl, Halogen.

In einer Ausführungsform sind R¹ und R² ausgewählt aus: -(C₁-C₁₂)-Alkyl, -O-(C₁-C₁₂)-Alkyl, - (C₆-C₂₀)-Aryl, -O-(C₆-C₂₀)-Aryl.

In einer Ausführungsform sind R¹ und R² ausgewählt aus: -(C₁-C₁₂)-Alkyl, -(C₆-C₂₀)-Aryl.

In einer Ausführungsform stehen R¹ und R² für -(C₁-C₁₂)-Alkyl.

In einer Ausführungsform stehen R¹ und R² für -(C₆-C₂₀)-Aryl.

In einer Ausführungsform stehen R¹ und R² für den gleichen Rest.

In einer Ausführungsform weist die Verbindung (**I**) die folgende Struktur auf:

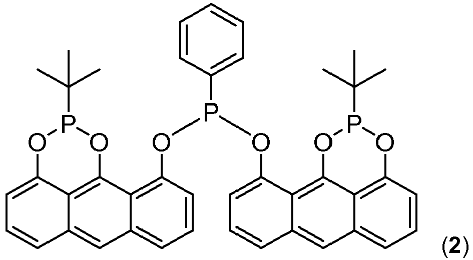

In einer Ausführungsform weist die Verbindung (**I**) die folgende Struktur auf:

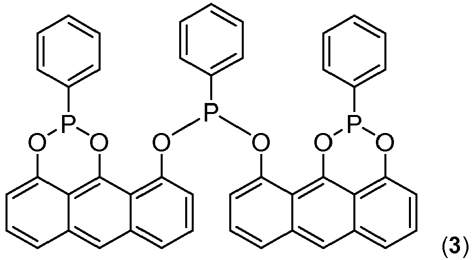

Neben der Verbindung an sich wird auch deren Verwendung in einem Ligand-Metall-Komplex zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Des Weiteren wird auch ein Verfahren beansprucht, in welchem eine zuvor beschriebene Verbindung eingesetzt wird.

Verfahren umfassend die Verfahrensschritte:
a) Vorlegen eines Olefins,
b) Zugabe eines Komplexes, welcher eine zuvor beschriebene Verbindung umfasst, sowie ein Metall ausgewählt aus: Rh, Ru, Co, Ir,
   oder einer zuvor beschriebene Verbindung und einer Substanz, welche ein Metall ausgewählt aus: Rh, Ru, Co, Ir aufweist;
c) Zuführen von H₂ und CO,
d) Erwärmen des Reaktionsgemisches, wobei das Olefin zu einem Aldehyd umgesetzt wird.

In einer Variante des Verfahrens ist das Metall Rh.

Im Folgenden wird die Erfindung anhand von Ausführungsbeispielen näher erläutert.

### Ligandensynthese

### Ligand 1: Bis(2-(tert.-butyl)anthrar[1,9-de][1,3,2]dioxaphosphinin-11-yl)tert.-butylphosphonit (Vergleichsligand)

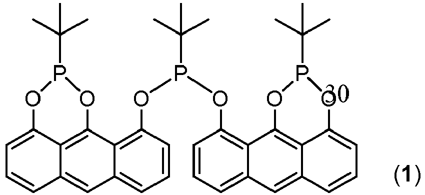

Zu einer Mischung aus 2-(*terl*.-Butyl)anthra[1,9-*de*][1,3,2]dioxaphosphinin-11-ol (0,306 g; 0,978 mmol) und Triethylamin (0,15 ml) in Toluol (3 ml) wird bei Raumtemperatur eine Lösung von *tert.-*Butyldichlorphosphin (0,233 g; 1,467 mmol) in Toluol (3 ml) getropft. Man rührt über Nacht und anschließend 4 h bei 70 °C, filtriert, engt das Filtrat im Vakuum ein und trocknet den viskosen Rückstand 6 h bei 40°C/ 0,1 mbar. Ausbeute: 0,418 g (0,588 mmol; 60%).
Elementaranalyse (ber. für C₄₀H₄₁O₆P₃= 710,68 g/mol): C 67,44 (67,60); H 6,34 (5,81); P 13,23 (13,08)%.
³¹P-NMR (CD₂Cl₂): 171,0 (s) + 203,6 (s) ppm; Diastereomer 1. 171,4 (s) + 205,3 (s) ppm; Diastereomer 2. Dia¹/Dia²=2:1.
¹H-NMR (CD₂Cl₂): 0,91-0,98 (m, 9H); 1,42 (d, ²*J*_{CP}= 14,3 Hz; 18 H); 6,92-6,96 (m, 2H); 7,25-7,29 (m, 1H); 7,36-7,43 (m, 5H); 7,58-7,62 (m, 2H); 7,67-7,74 (m, 2H); 8,05 (s, br, 2H) ppm.
¹³C NMR (CD₂Cl₂): 23,2; 23,4; 23,5; 23,8; 23,9; 24,0; 24,1; 24,2; 24,4; 37,4 (d, ; 37,9; 39,0; 39,3; 39,6; 39,8; 110,1; 110,3; 113,0; 113,3; 114,0; 114,8; 115,1; 116,9; 119,7; 119,9; 120,1; 122,0; 122,1; 124,6; 124,9; 125,8; 125,9; 126,0; 127,1; 127,2; 132,8; 135,1; 144,2; 147,2; 152,9 ppm. ESI-TOF HRMS: *m*/*z* 711,21768 [M⁺+H], ber.: *m*/*z* 711,21887.

### Ligand 2: Bis(2-(tert.-butyl)anthra[19-de][1,3,2]dioxaphosphinin-11-yl)phenylphosphonit

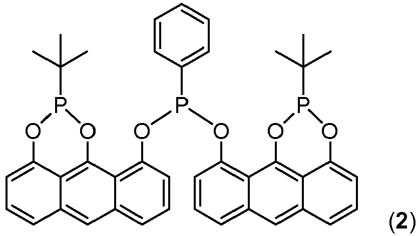

Zu einer Mischung aus 2-(*tert*.-Butyl)anthra[1,9-*de*][1,3,2]dioxaphosphinin-11-ol (0,651 g; 2,084 mmol) und Triethylamin (0,32 ml) in Toluol (7 ml) wird bei Raumtemperatur eine Lösung von Phenyldichlorphosphin (0,187 g; 1,042 mmol) in Toluol (4 ml) getropft. Man rührt über Nacht und anschließend 4 h bei 70 °C, filtriert, engt das Filtrat im Vakuum ein und trocknet den orangefarbenen Rückstand 2 h bei 40°C/ 0,1 mbar. Der erhaltene Feststoff wird mit Heptan (12 ml) verrührt, abfiltriert und im Vakuum bei 40 °C getrocknet. Ausbeute: 0,390 g (0,533 mmol; 26%).
Elementaranalyse (ber. für C₄₂H₃₇O₆P₃= 730,67 g/mol): C 69,09 (69,04); H 5,11 (5,10); P 12,45 (12,72)%.
³¹P-NMR (CD₂Cl₂): 153,8 (s) + 175,2 (s) ppm; Diastereomer 1. 157,8 (s) + 174,7 (s) + 175,5 (s) ppm; Diastereomer 2. Dia¹/Dia²=1:8.
¹H-NMR (CD₂Cl₂): 0,89-0,99 (m, 18H); 6,92-6,98 (m, 2H); 7,19-7,33 (m, 4H); 7,35-7,51 (m, 5H); 7,58-7,69 (m, 4H); 8,02-8,14 (m, 4H) ppm.
¹³C NMR (CD₂Cl₂): 23,1; 23,4; 37,5 (d, *J*_{CP}= 33,8 Hz); 37,7 (d, *J*_{CP}= 34,8 Hz); 110,2; 110,3; 114,1; 114,3; 114,5; 115,3; 115,5; 119,8; 119,9; 121,9; 122,0; 123,9; 124,3; 126,1; 126,2; 126,9; 128,6; 128,7; 131,3; 131,6; 132,9; 135,1; 139,6; 139,8; 145,2; 145,3; 148,0; 148,1; 152,5; 152,7 ppm. ESI-TOF HRMS: *m*/*z* 753,16955 [M⁺+Na], ber.: *m*/*z* 753,16955.

### Ligand 3: Bis(2-phenylanthra[1,9-de][1,3,2]dioxaphosphinin-11-yl) phenylphosphonit

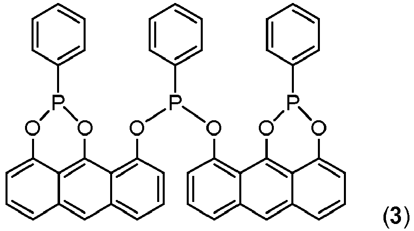

### a) Vorstufe: 2-Phenylanthra[1,9-de][1,3,2]dioxaphosphinin-11-ol

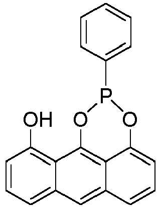

Zu einer Mischung aus Anthracentriol (0,932 g; 4,118 mmol) und Triethylamin (1,26 ml) in Toluol (20 ml) wird bei Raumtemperatur eine Lösung von Phenyldichlorphosphin (0,737 g; 4,118 mmol) in Toluol (10 ml) getropft. Man rührt über Nacht und anschließend 3 h bei 70 °C, setzt Toluol zu (8 ml) und filtriert. Der Filterkuchen wird Toluol (3x5 ml) gewaschen und die vereinigten Filtrate werden im Vakuum eingeengt. Der erhaltene Feststoff wird zunächst 2 h bei 40°C/0,1 mbar behandelt und dann mit Toluol (12 ml) für 1h verrührt. Filtration und Trocknung im Vakuum bei 40 °C ergeben 0,729 g (2,193 mmol; 53%).
Elementaranalyse (ber. für C₂₀H₁₃O₃P= 332,29 g/mol): C 72,11 (72,29); H 3,97 (3,94); P 9,23 (9,32)%.
³¹P-NMR (CD₂Cl₂): 151,1 (s) ppm.
¹H-NMR (CD₂Cl₂): 6,92 (dd, *J*^{A}= 7,0 Hz; *J*^{B}= 1,3 Hz; 1H); 7,07 (dt, *J*^{A}= 7,3 Hz; *J*^{B}= 1,0 Hz; 1H); 7,17-7,26 (m, 3H); 7,31-7,57 (m, 6H); 8,0 (s, 1H); 9,05 (s, 1H) ppm.
¹³C NMR (CD₂Cl₂): 109,2; 111,8; 113,0; 115,6; 119,2; 119,9; 121,2; 123,1; 126.4; 127,8; 129,1; 129,2; 129,2; 129,5; 130,9; 132,6; 134,5; 136,6; 139,9; 140,4; 143,2; 145.3; 153,9 ppm.
EI HRMS: *m*/*z* 332,05937 [M⁺], ber.: *m*/*z* 332,05968.

### b) Ligand (3)

Zu einer Mischung aus 2-Phenylanthra[1,9-*de*][1,3,2]dioxaphosphinin-11-ol (0,350 g; 1,053 mmol) und Triethylamin (0,16 ml) in Toluol (11 ml) wird bei Raumtemperatur eine Lösung von Phenyldichlorphosphin (0,094 g; 0,526 mmol) in Toluol (10 ml) getropft. Man rührt über Nacht und anschließend 4 h bei 70 °C und filtriert. Das Filtrat wird im Vakuum eingeengt und der erhaltene gelbe Feststoff bei 40°C/0,1 mbar getrocknet. Ausbeute: 0,265 g (0,344 mmol; 33%).
Elementaranalyse (ber. für C₄₆H₂₉O₆P₃= 770,65 g/mol): C 71,74 (71,69); H 3,89 (3,79)%. ³¹P-NMR (CD₂Cl₂): 144,5 (s) + 144,9 + 159,9 (s) ppm; Diastereomer 1. 145,0(s) + 157,0 (s) ppm; Diastereomer 2. Dia¹/Dia²=1:1.
¹H-NMR (CD₂Cl₂): 6,82-7,51 (m, 25H); 7,77-7,82 (m, 2H); 8,08-8,19 (m, 2H) ppm.
¹³C NMR (CD₂Cl₂) δ 111,6; 114,4; 114,7; 115,1; 115,3; 115,9; 116,1; 116,2; 120,6; 120,8; 122,6; 123,9; 124,3; 124,7; 125,6; 126,2; 126,3; 126,6; 128,6; 128,6; 128,7; 128,8; 128,9; 129,3; 129,4; 129,4; 129,5; 129,6; 129,7; 130,1; 130,2; 131,1; 131,4; 131,5; 131,6; 131,7; 132,9; 134,8; 138,4; 141,2; 141,8; 143,6; 143,7; 146,5; 152,1; 152,3 ppm.
ESI-TOF HRMS: *m*/*z* 841,09195 [M⁺+ 3/2 O₂ + Na], ber.: *m*/*z* 841,09166.

### Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol in einem Pure Solv. MD-7 System gereinigt und unter Argon aufbewahrt.

Im Autoklaven wurden unter Argonatmosphäre Lösungen der Katalysatorvorstufe und des Liganden gemischt. Als Katalysatorvorstufe kam [(acac)Rh(COD)] (Umicore, acac = Acetylacetonat-Anion; COD = 1,5-Cyclooctadien) zum Einsatz. Von der Rhodiumverbindung wurden 10 ml einer 4,31 millimolaren Lösung in den Autoklaven gegeben. Anschließend wurde die entsprechende Menge an Liganden (L/Rh = 1:1) in 10 ml Toluol gelöst und zugemischt. Durch Zugabe von weiterem Toluol wurde das Anfangsvolumen der Katalysatorlösung auf 41,0 ml eingestellt. In eine druckfeste Pipette wurde 1-Octen eingefüllt. Der Autoklav wurde bei einem Gesamtgasdruck (Synthesegas: Linde; H₂ (99,999%) : CO (99,997%) = 1:1) von 42 bar aufgeheizt. Nach Erreichen der Reaktionstemperatur von 100 °C wurde der Synthesegasdruck auf 48,5 bar erhöht und das Olefin mit einem in der Druckpipette eingestellten Überdruck von ca. 3 bar in den Autoklaven gepresst. Die Reaktion wurde bei konstantem Druck von 50 bar (Nachdruckregler der Fa. Bronkhorst, NL) über 4 h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 10 ml Pentan verdünnt und gaschromatographisch analysiert: HP 5890 Series II plus, PONA, 50 m x 0,2 mm x 0,5 µm.

Reaktionsbedingungen: 1-Octen, T = 100 °C, p = 50 bar CO/H₂, t = 4 h, [Rh] = 0,77 mM, Rh/Olefin = 1:2210, Verhältnis L/Rh = 1:1, Lösungsmittel: Toluol.

### Ergebnisse der Katalyseversuche

**Tabelle**

| Ligand | Gesamtausbeute Aldehyd (%) |
|---|---|
| **1** | 29,2 |
| **2*** | 94,7 |
| **3*** | 97,5 |

| | |
|---|---|
| * erfindungsgemäße Verbindung | |

Wie die oben beschriebenen Versuche zeigen, wird die Aufgabe durch einen erfindungsgemäßen Liganden gelöst.

## Patentansprüche

1. Verbindung der Formel (**I**): 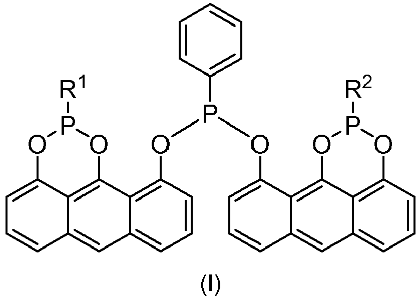 wobei
R¹, R² ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl;
wobei die genannten Alkylgruppen und Arylgruppen wie folgt substituiert sein können: substituierte -(C₁-C₁₂)-Alkylgruppen, substituierte -O-(C₁-C₁₂)-Alkyl, substituierte -(C₆-C₂₀)-Aryl, substituierte -O-(C₆-C₂₀)-Aryl können einen oder mehrere Substituenten aufweisen; die Substituenten sind unabhängig voneinander ausgewählt aus: -(C₁-C₁₂)-Alkyl, -O-(C₁-C₁₂)-Alkyl, Halogen.

2. Verbindung nach Anspruch 1,
wobei R¹ und R² ausgewählt sind aus: -(C₁-C₁₂)-Alkyl, -O-(C₁-C₁₂)-Alkyl, -(C₆-C₂₀)-Aryl, -O-(C₆-C₂₀)-Aryl.

3. Verbindung nach einem der Ansprüche 1 oder 2,
wobei R¹ und R² ausgewählt sind aus: -(C₁-C₁₂)-Alkyl, -(C₆-C₂₀)-Aryl.

4. Verbindung nach einem der Ansprüche 1 bis 3,
wobei R¹ und R² für -(C₁-C₁₂)-Alkyl stehen.

5. Verbindung nach einem der Ansprüche 1 bis 4,
wobei R¹ und R² für -(C₆-C₂₀)-Aryl stehen.

6. Verbindung nach einem der Ansprüche 1 bis 5,
wobei R¹ und R² für den gleichen Rest stehen.

7. Verwendung einer Verbindung nach Anspruch 1 bis 6,
in einem Ligand-Metall-Komplex zur Katalyse einer Hydroformylierungsreaktion.

8. Verfahren umfassend die Verfahrensschritte:
a) Vorlegen eines Olefins,
b) Zugabe eines Komplexes, welcher eine Verbindung gemäß den Ansprüchen 1 bis 6 umfasst, sowie ein Metall ausgewählt aus: Rh, Ru, Co, Ir,
oder einer Verbindung nach einem der Ansprüche 1 bis 6 und einer Substanz, welche ein Metall ausgewählt aus: Rh, Ru, Co, Ir aufweist;
c) Zuführen von H₂ und CO,
d) Erwärmen des Reaktionsgemisches, wobei das Olefin zu einem Aldehyd umgesetzt wird.

## Claims

1. Compound of formula (I): 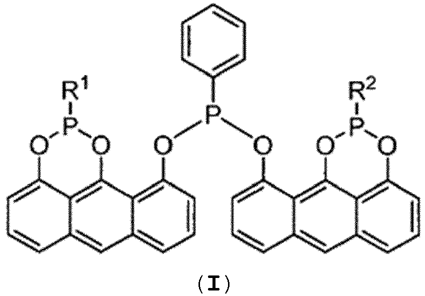 where
R¹, R² are selected from -H, -(C₁-C₁₂) alkyl, -O-(C₁-C₁₂) alkyl, -(C₆-C₂₀) aryl, -O-(C₆-C₂₀) aryl;
wherein the named alkyl moieties and aryl moieties may be substituted as follows: substituted -(C₁-C₁₂) alkyl moieties, substituted -O-(C₁-C₁₂) alkyl, substituted -(C₆-C₂₀) aryl, substituted -O-(C₆-C₂₀) aryl may have one or more substituents; the substituents are independently selected from: -(C₁-C₁₂) alkyl, -O-(C₁-C₁₂) alkyl, halogen.

2. Compound according to Claim 1,
where R¹ and R² are selected from -(C₁-C₁₂) alkyl, -O-(C₁-C₁₂) alkyl, -(C₆-C₂₀) aryl, -O-(C₆-C₂₀) aryl.

3. Compound according to either of Claims 1 and 2,
where R¹ and R² are selected from -(C₁-C₁₂) alkyl, -(C₆-C₂₀) aryl.

4. Compound according to any of Claims 1 to 3,
where R¹ and R² represent -(C₁-C₁₂) alkyl.

5. Compound according to any of Claims 1 to 4,
where R¹ and R² represent -(C₆-C₂₀) aryl.

6. Compound according to any of Claims 1 to 5,
where R¹ and R² represent the same radical.

7. Use of a compound according to Claims 1 to 6
in a ligand-metal complex for catalysis of a hydroformylation reaction.

8. Process comprising the following process steps:
a) starting with an olefin,
b) adding a complex that comprises a compound according to Claims 1 to 6,
and a metal selected from Rh, Ru, Co, Ir,
or a compound according to any of Claims 1 to 6 and a substance that contains a metal selected from Rh, Ru, Co, Ir;
c) feeding in H₂ and CO,
d) heating the reaction mixture, wherein the olefin is converted into an aldehyde.

## Revendications

1. Composé de la formule (I) : 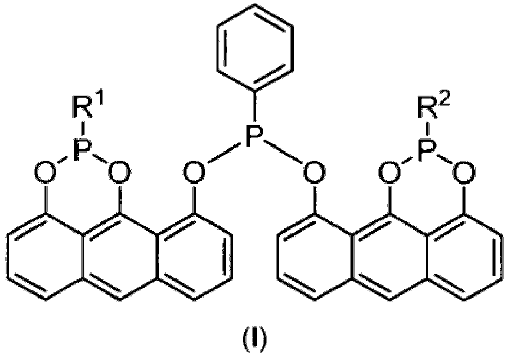 dans laquelle
R¹, R² sont choisis parmi : H, alkyle en (C₁-C₁₂), -O-alkyle en (C₁-C₁₂), aryle en (C₆-C₂₀), -O-aryle en (C₆-C₂₀);
les groupes alkyle et les groupes aryle mentionnés pouvant être substitués de la manière suivante :
les groupes alkyle en (C₁-C₁₂) substitués, les groupes - O-alkyle en (C₁-C₁₂) substitués, les groupes aryle en (C₆-C₂₀) substitués, les groupes -O-aryle en (C₆-C₂₀) substitués peuvent comprendre un ou plusieurs substituants ; les substituants sont choisis indépendamment les uns des autres parmi : alkyle en (C₁-C₁₂), -O-alkyle en (C₁-C₁₂), halogène.

2. Composé selon la revendication 1, dans lequel R¹ et R² sont choisis parmi : alkyle en (C₁-C₁₂), -O-alkyle en (C₁-C₁₂), aryle en (C₆-C₂₀), -O-aryle en (C₆-C₂₀).

3. Composé selon l'une quelconque des revendications 1 ou 2, dans lequel R¹ et R² sont choisis parmi : alkyle en (C₁-C₁₂), aryle en (C₆-C₂₀).

4. Composé selon l'une quelconque des revendications 1 à 3, dans lequel R¹ et R² représentent alkyle en (C₁-C₁₂).

5. Composé selon l'une quelconque des revendications 1 à 4, dans lequel R¹ et R² représentent aryle en (C₆-C₂₀).

6. Composé selon l'une quelconque des revendications 1 à 5, dans lequel R¹ et R² représentent le même radical.

7. Utilisation d'un composé selon les revendications 1 à 6 dans un complexe ligand-métal pour la catalyse d'une réaction d'hydroformylation.

8. Procédé comprenant les étapes de procédé suivantes :
a) le chargement d'une oléfine,
b) l'ajout d'un complexe qui comprend un composé selon les revendications 1 à 6, ainsi qu'un métal choisi parmi : Rh, Ru, Co, Ir,
ou d'un composé selon l'une quelconque des revendications 1 à 6 et d'une substance qui comprend un métal choisi parmi : Rh, Ru, Co, Ir ;
c) l'introduction d'H₂ et de CO,
d) le chauffage du mélange réactionnel, l'oléfine étant transformée en un aldéhyde.
